(19) Europäisches Patentamt
European Patent Office
Office européen des brevets

(11) Publication number: **0 211 457 A2**

(12) **EUROPEAN PATENT APPLICATION**

(21) Application number: **86201203.6**

(22) Date of filing: **09.07.86**

(51) Int. Cl.[4]: **C07D 237/20** , C07D 237/22 , C07D 403/04 , C07D 409/12 , C07D 409/14 , A61K 31/50

(30) Priority: **31.07.85 US 760845**

(43) Date of publication of application: **25.02.87 Bulletin 87/09**

(84) Designated Contracting States: **AT BE CH DE FR GB IT LI LU NL SE**

(71) Applicant: **JANSSEN PHARMACEUTICA N.V.**
**Turnhoutseweg 30**
**B-2340 Beerse(BE)**

(72) Inventor: **Stokbroekx, Raymond A.**
**Rode Kruisstraat 13**
**B-2340 Beerse(BE)**

(54) **Novel (4-substituted-piperazinyl)pyridazines.**

(57) Novel (4-substituted-piperazinyl)pyridazines of formula

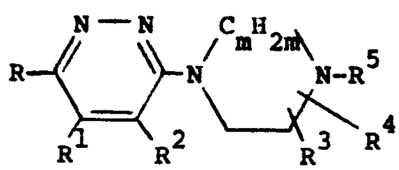 (I),

wherein R is hydrogen, halo, $C_{1-6}$ alkyloxy, hydroxy or phenyl;

m is the integer 2 or 3;

$R^1$, $R^2$, $R^3$ and $R^4$ are, each independently, hydrogen, or $C_{1-6}$alkyl;

or where $R^3$ and $R^4$ are substituted on a different carbon atom, $R^3$ and $R^4$ taken together, may form a bivalent radical $-CH_2-CH_2-$;

$R^5$ is (aryl)$C_{2-6}$alkenyl, (aryl)$C_{2-6}$alkynyl, (aryl)-(hydroxy)$C_{1-6}$alkyl or (aryl)(oxo)$C_{1-6}$ alkyl;

the N-oxide forms, the pharmaceutically acceptable acid addition salts and the possible stereochemically isomeric forms, which compounds are analgesic and antitussive agents; pharmaceutical compositions containing such compounds as an active ingredient and methods of preparing said compounds and pharmaceutical compositions.

EP 0 211 457 A2

# NOVEL (4-SUBSTITUTED-PIPERAZINYL)PYRIDAZINES

Background of the invention:

In J. Med. Chem. 24, 59-63 (1981) there are described a number of 1H-imidazolyl-pyridazines, while in Published European Patent Application No. 55,583, U.S. Patent Nos. 4,110,450, 4,104,385 and 2,985,657 a number of piperazinyl, pyrrolidinyl and piperidinyl substituted pyridazines are described as intermediates. In Published European Patent Application No. 9,655 3-chloro-6-[4-(2-methoxyphenyl)-1-piperazinyl]pyridazine and 1-chloro-4-(4-hydroxypiperidino)-phtalazine are also described as intermediates.

Moreover a number of substituted 1-piperazinyl-pyridazines are described in J. Med. Chem. 6, 541-4 (1963), in ibid. 8, 104-107 (1965) and ibid. 15, 295-301 (1972) as compounds having adrenolytic, antihistaminic or analgesic activity.

The compounds of the present invention differ from the cited prior-art compounds by the specific substitution on the pyridazine moiety and particularly by their useful analgetic and antitussive properties.

Description of the Invention:

The present invention is concerned with novel pyridazinamines which may structurally be represented by the formula

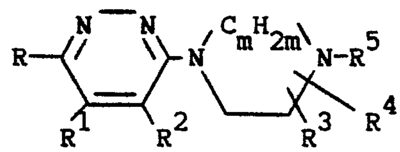 (I),

the N-oxide forms, the pharmaceutically acceptable acid addition salts and the possible stereochemically isomeric forms thereof, wherein R is hydrogen, halo, $C_{1-6}$alkyloxy, hydroxy or phenyl;

m is the integer 2 or 3;

$R^1$, $R^2$, $R^3$ and $R^4$ are, each independently, hydrogen or $C_{1-6}$alkyl;

or where $R^3$ and $R^4$ are substituted on a different carbon atom, $R^3$ and $R^4$ taken together, may form a bivalent radical $-CH_2-CH_2-$;

$R^5$ is (aryl)$C_{2-6}$alkenyl, (aryl)$C_{2-6}$alkynyl, (aryl)-(hydroxy)-$C_{1-6}$alkyl or (aryl)(oxo)$C_{1-6}$ alkyl;

wherein aryl is phenyl, being optionally substituted with up to 3 substituents, each independently selected from the group consisting of halo, $C_{1-6}$alkyl, trifluoromethyl, nitro, amino, $C_{1-6}$ alkyloxy, hydroxy and $C_{1-6}$alkyloxycarbonyl; thienyl; and naphthalenyl.

As used in the foregoing definitions the term halo is generic to fluoro, chloro, bromo and iodo; "$C_{1-6}$alkyl" is meant to include straight and branched saturated hydrocarbon radicals, having from 1 to 6 carbon atoms, such as, for example, methyl, ethyl, 1-methylethyl, 1,1-dimethylethyl, propyl, butyl, pentyl, hexyl and the like;

"$C_{2-6}$alkenyl " refers to alkenyl radicals having from 2 to 6 carbon atoms, such as, for example, 2-propenyl, 2-butenyl, 3-butenyl, 2-pentenyl and the like; "$C_{2-6}$alkynyl" refers to alkynyl radicals having from 2 to 6 carbon atoms such as, for example, 2-propynyl, 2-butynyl, 3-butynyl, 2-pentynyl and the like.

It is evident that $R^3$ and/or $R^4$ may be substituted on the same carbon atom or on different carbon atoms of either the radical $C_mH_{2m}$or $CH_2$-$CH_2$ bridging the two nitrogen atoms of the saturated ring moiety.

The said N-oxides of the compounds of formula (I) are meant to comprise those compounds of formula (I) wherein one or several nitrogen atoms are oxidated to the so-called N-oxide form. Preferred N-oxides are those containing only one oxidated nitrogen which is situated in the saturated ring system bearing $R^3$,$R^4$ and $R^5$.

Preferred compounds within the invention are those wherein R is halo, $R^5$ is 3-aryl-2-propenyl, $R^1$, $R^2$, $R^3$ and $R^4$ are hydrogen and m is 2.

The most preferred compounds within the invention are selected from the group consisting of 3-chloro-6-[4-(3-phenyl-2-propenyl)-1-piperazinyl]-pyridazine, the pharmaceutically acceptable acid addition salts and possible stereochemically isomeric forms thereof.

The compounds of formula (I) can generally be prepared by reacting an amine of formula (II) with a reagent of formula (III) following art-known N-alkylation procedures.

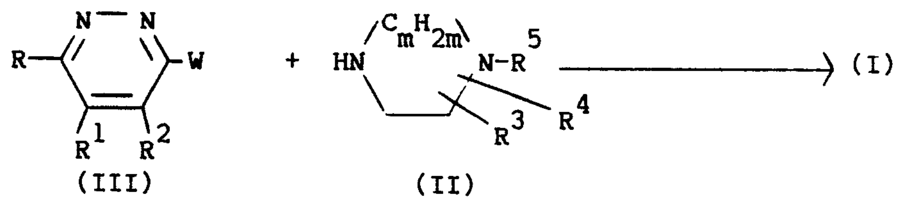

In (III) W represents an appropriate reactive leaving group such as, for example, halo, i.e. fluoro, chloro, bromo or iodo, or a sulfonyloxy group, e.g. methylsulfonyloxy or 4-methylphenylsulfonyloxy, a $C_{1-6}$alkyloxy or $C_{1-6}$alkylthio group.

The reaction of (II) with (III) can conveniently be conducted in an inert organic solvent such as, for example, an aromatic hydrocarbon, e.g., benzene, methylbenzene, dimethylbenzene, and the like; a lower alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran and the like; a dipolar aprotic solvent such as, for example, N,N-dimethylformamide (DMF); N,N-dimethylacetamide (DMA); dimethyl sulfoxide (DMSO); nitrobenzene; 1-methyl-2-pyrrolidinone; and the like. The addition of an appropriate base such as, for example, an alkali metal carbonate or hydrogen carbonate, sodium hydride or an organic base such as, for example, N,N-diethylethanamine or N-(1-methylethyl)-2-propanamine may be utilized to pick up the acid which is liberated during the course of the reaction. In some circumstances the addition of a iodide salt, preferably an alkali metal iodide, is appropriate. The alkylation reactions can also be conducted by mixing and/or melting the reactants together, optionally in the presence of the bases mentioned hereinabove. Somewhat elevated temperatures may be used to enhance the rate of the reaction.

The compounds of formula (I) can also be prepared by N-alkylating an amine of formula (IV) with a reagent of formula (V) following the same procedures described hereinabove for the preparation of (I) starting from (II) and (III).

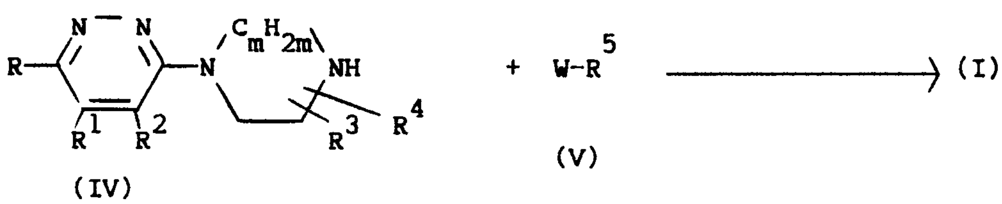

In (V) W has the previously defined meaning.

The compounds of formula (I) wherein $R^5$ is 3-aryl-3-oxo-propyl can also be prepared by reacting an appropriate 1-aryl-ethanone with a piperazine of formula (IV) in the presence of formaldehyde or a polymeric form thereof in a suitable solvent such as, for example, an alcohol, e.g. methanol, ethanol, 2-propanol and the like.

The compounds of formula (I) can also be converted into each other by an appropriate groupstransformation reaction. For example, the compounds of formula (I) wherein $R^5$ is (aryl)(oxo)-$C_{1-6}$alkyl can be converted into compounds of formula (I) wherein $R^2$ is (aryl)(hydroxy)$C_{1-6}$alkyl by an appropriate reduction reaction, e.g. by treating the starting compounds with a complex metal hydride, e.g. sodium borohydride, in a suitable solvent such as, for example, an alcohol, e.g. methanol and ethanol.

The compounds of formula (I) wherein $R^5$ is -(aryl)$C_{2-6}$alkynyl can be converted into compounds of formula (I) wherein $R^5$ is (aryl)$C_{2-6}$alkenyl following art-known procedures for carrying out a reduction reaction, e.g. by treating the starting compounds with hydrogen in the presence of a suitable catalyst such as, for example, platinium-on-charcoal, palladium-on-charcoal and the like catalysts.

The compounds of formula (I) wherein $R^5$ is -(aryl)(hydroxy)$C_{1-6}$alkyl can be converted into the corresponding compounds of formula (I) wherein $R^5$ is (aryl)$C_{2-6}$alkenyl by an appropriate elimination reaction. This can be accomplished by reacting the former compounds with a suitable acidic solution preferably at higher temperatures.

Suitable acidic solutions contain one or more acids such as sulfuric, hydrochloric, acetic and the like acids in admixture with water and/or an organic solvent, such as methanol, ethanol and the like.

Or, the starting hydroxy containing compounds can be reacted with an appropriate dehydrating agent such as, for example, phosphoryl chloride, thionyl chloride, phosphor trichloride, preferably in the presence of a suitable solvent such as, for example, pyridine, N,N -dimethylformamide (DMF) and the like.

The compounds of formula (I) wherein R is halo may be converted into the corresponding compounds wherein R is $C_{1-6}$alkyloxy by reacting the starting compounds with an appropriate alkalimetal alkoxide in a suitable solvent, preferably in the corresponding alcohol. Furthermore, the compounds of formula (I) wherein R is halo can be converted to the corresponding compounds of formula (I) wherein R is hydroxy by an appropriate hydrolysis reaction, e.g. by treating the starting materials with a basic aqueous solution.

The compounds of formula (I), wherein R is halo may be converted into the corresponding compounds wherein R is hydrogen, following art-known hydrogenolysis procedures, i.e. by heating the starting compounds in a suitable solvent under hydrogen atmosphere in the presence of an appropriate catalyst, e.g. palladium-on-charcoal and the like catalysts.

The compounds of formula (I) can be converted to the corresponding N-oxide forms following art-known procedures for converting a trivalent nitrogen to its N-oxide-form. SuchN-oxidation reaction may generally be carried out by reacting the starting compound with an appropriate organic or inorganic peroxide. Appropriate inorganic peroxides can, for example, be hydrogen peroxide, an alkali metal or earth alkaline metal peroxide, e.g. sodium peroxide, potassium peroxide, barium peroxide and the like; appropriate organic peroxides may comprise peroxy acids such as, for example, benzenecarboperoxoic acid or halo substituted benzenecarboperoxoic acid, e.g. 3-chlorobenzenecarboperoxoic acid and the like, peroxoalkanoic acids, e.g. peroxoacetic acid and the like, alkylhydroperoxides, e.g. t.butyl hydroperoxide and the like.

Suitable solvents are, for example, water, lower alkanols, e.g. methanol, ethanol, propanol, butanol and the like, hydrocarbons, e.g. benzene, methylbenzene, dimethylbenzene and the like, ketones, e.g. 2-propanone, 2-butanone and the like, halogenated hydrocarbons, e.g. dichloromethane, trichloromethane and the like, and mixtures of such solvents. In order to enhance the reaction rate, it may be appropriate to heat the reaction mixture.

In all of the foregoing and in the following preparations, the reaction products may be isolated from the reaction mixture and, if necessary, further purified according to methodologies generally known in the art.

The compounds of formula (I) have basic properties and, consequently, they may be converted to their therapeutically active non-toxic acid-addition salt forms by treatment with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

A number of intermediates and starting materials in the foregoing preparations are known compounds which may be prepared according to art-known methodologies as described, for example, in Published European Patent Application No. 55,583 and U.S Patent No. 2,985,657.

The intermediates of formula (II) may be prepared by alkylating a cyclic amine of formula (VI) with a reagent of formula (V), thus preparing an intermediate of formula (VII), and subsequently eliminating the group P. In (VI) and (VII) P is an appropriate protective group such as, for example, $C_{1-6}$alkyloxycarbonyl, arylmethoxycarbonyl, arylmethyl, arylsulfonyl and the like. The elimination of P in (VII) may generally be carried out following art-known procedures such as, for example, by hydrolysis in alkaline or acidic medium.

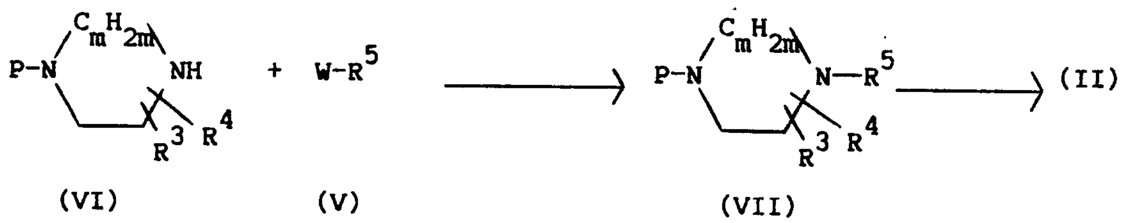

The intermediates of formula (IV) can conveniently be prepared by reacting a cyclic amine of formula (VI) with a pyridazine of formula (III) and subsequently eliminating the protective group in P in (VIII).

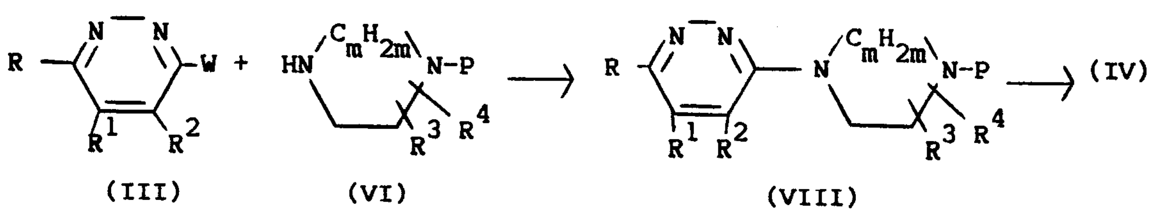

The compounds of this invention may have several asymmetric carbon atoms in their structure. Each of these chiral centers may be present in a R-and a S-configuration, this R-and S-notation being in correspondence with the rules described in J. Org. Chem., 35, 2849-2867 (1970).

Furthermore, the compounds of this invention may be present as E-or Z-forms, this E-and Z-notation being in correspondence with the rules also described in J. Org. Chem., 35, 2849-2867 - (1970).

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, and enantiomers may be separated from each other by the selective crystallization of their diastereomeric salts with optically active acids.

Pure stereochemically isomeric forms may also be derived from the corresponding pure stereochemically isomeric forms of the appropriate starting materials, provided that the reaction occurs stereospecifically.

Stereochemically isomeric forms of the compounds of formula (I) are naturally intended to be embraced within the scope of the invention.

The compounds of formula (I) and the pharmaceutically acceptable acid addition salts thereof have very interesting pharmacological properties. More particularly they are potent analgesics and as such they can be used to depress pain in warm-blooded animals. They also possess antitussive properties which makes them useful in the treatment of cough, and moreover they show anti-viral activity. The useful properties of the compounds of formula (I) and their pharmaceutically acceptable acid addition salts can be demonstrated by, for example, "The Tail Withdrawal Test" and "The Acetic Acid-Induced Writing in Rats Test".

Due to their useful pharmacological properties, the compounds of formula (I) and their acid-addition salts are very useful as analgesics and antitussives. In view of said useful pharmacological properties, the subject compounds may be formulated into various pharmaceutical forms for administration purposes.

To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches,

sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not introduce a significant deletorious effect on the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier.

Examples of such dosage unit forms are tablets (including scored or coated tablets), capsules, pills, powder packets, wafers, injectable solutions or suspensions, teaspoonfuls, tablespoonfuls and the like, and segregated multiples thereof.

In view of the analgesic activity of the subject compounds, it is evident that the present invention provides a method of preventing or combatting pain in warm-blooded animals. Said method comprises the systemic administration of an effective analgesic amount of a compound of formula (I) a N-oxide form, a pharmaceutically acceptable acid addition salt or a stereochemically isomeric form thereof in admixture with a pharmaceutical carrier.

Those of skill in preventing or combatting pain in warm-blooded animals could easily determine the effective amount from the test results presented hereinafter.

Although the amount of active ingredient to be administered may vary within rather wide limits, depending on the particular circumstances of the case, doses from about 0.1 mg/kg body weight to about 250 mg/kg body weight and preferably from 0.5 mg/kg to 50 mg/kg body weight administered once or repeatedly are generally found effective.

The following examples are intended to illustrate and not to limit the scope of the present invention in all its aspects. Unless otherwise stated all parts therein are by weight.

## EXPERIMENTAL PART

### A. Preparation of Intermediates

#### Example 1

To a stirred solution of 300 parts of hexahydro-1H-1,4-diazepine in 900 parts of methylbenzene were added 75 parts of 3,6-dichloropyridazine. The whole was stirred and refluxed for 4 hours. The reaction mixture was evaporated. Water was added to the residue. The product was extracted with trichloromethane. The extract was dried, filtered and evaporated. The residue was converted into the hydrochloride salt in 2-propanol and ethanol. The salt was filtered off and dried, yielding 28 parts (22%) of 1-(6-chloro-3-pyridazinyl)-hexahydro-1H-1,4-diazepine monohydrochloride (1).

In a similar manner there were also prepared:

ethyl 4-(6-chloro-5-methyl-3-pyridazinyl)-1-piperazinecarboxylate;

mp. 132.2°C (2);

hexahydro-1-(6-phenyl-3-pyridazinyl)-1H-1,4-diazepine as a residue (3);

3-chloro-6-(3-methyl-1-piperazinyl)pyridazine; mp. 78.6°C (4);

cis-(E)-2,5-dimethyl-1-(3-phenyl-2-propenyl)-piperazine as a residue (5); and

3-chloro-6-(3,5-dimethyl-1-piperazinyl)pyridazine - (6).

Example 2

A mixture of 22 parts of ethyl 4-(6-chloro-5-methyl-3-pyridazinyl)-1-piperazinecarboxylate, 28 parts of potassium hydroxide and 160 parts of 1-butanol was stirred overnight at reflux temperature. The reaction mixture was evaporated. Water was added. The product was extracted with trichloromethane. The extract was dried, filtered and evaporated. 2.2'-Oxybispropane was added. The product was filtered off and dried, yielding 17 parts (100%) of 3-chloro-4-methyl-6-(1-piperazinyl)-pyridazine (7).

Example 3

A mixture of 15.3 parts of (E)-(3-chloro-1-propenyl)benzene, 12 parts of 2-methylpiperazine, 28.5 parts of sodium carbonate and 225 parts of N,N-dimethylformamide was stirred overnight at 65°C. The reaction mixture was poured into ice water and the product was extracted with dichloromethane. The extract was dried, filtered and evaporated. The residue was purified by column chromatography over silica gel using a mixture of trichloromethane and methanol (96:4 by volume) as eluent. The pure fractions were collected and the eluent was evaporated. The residue was converted into the hydrochloride salt in 2-propanol. The salt was filtered off and dried, yielding 8.27 parts - (28.6%) of (E)-3-methyl-1-(3-phenyl-2-propenyl)-piperazine dihydrochloride; mp. 202°C (8).

B. Preparation of Final compounds

Example 4

A mixture of 3.8 parts of 3,6-dichloropyridazine, 4.1 parts of 1-(3-phenyl-2-propenyl)piperazine, 6.4 parts of sodium carbonate and 180 parts of N,N-dimethylformamide was stirred overnight at 65°C. The reaction mixture was poured into ice water. The product was filtered off and dissolved in dichloromethane. The organic layer was washed with water, dried, filtered and evaporated. The residue was purified by column chromatography over silica gel using a mixture of trichloromethane and methanol (98:2 by volume) as eluent. The pure fractions were collected and the eluent was evaporated. The residue was crystallized from 2-propanol. The product was filtered off and dried, yielding 3.7 parts - (58.8%) of (E)-3-chloro-6-[4-(3-phenyl-2-propenyl)-1-piperazinyl]pyridazine; mp. 129.0°C (1).

In a similar manner there was also prepared:

(E)-3-chloro-6-[2-methyl-4-(3-phenyl-2-propenyl)-1-piperazinyl]pyridazine monohydrochloride.hemihydrate; mp.138.4°C (2).

Example 5

A mixture of 6 parts of 3,6-dibromopyridazine, 4.1 parts of 1-(3-phenyl-2-propenyl)piperazine, 6.4 parts of sodium carbonate and 180 parts of N,N-dimethylacetamide was stirred overnight at 65°C. The reaction mixture was poured into ice water. The product was filtered off and dissolved in dichloromethane. The solution was washed with water, dried, filtered and evaporated. The residue was crystallized from a mixture of ethanol and 2-propanol. The product was filtered off and dried, yielding 2.9 parts (40.3%) of (E)-3-bromo-6-[4-(3-phenyl-2-propenyl)-1-piperazinyl]pyridazine; mp. 134.6°C (3).

Example 6

A mixture of 3 parts of (3-chloro-1-propenyl)-benzene, 3 parts of 3-methoxy-6-(1-piperazinyl)-pyridazine, 3.2 parts of sodium carbonate and 90 parts of N,N-dimethylacetamide was stirred overnight at 60°C. The reaction mixture was poured into water. The product was filtered off and taken up in trichloromethane. The solution was purified by column chromatography over silica gel using a mixture of trichloromethane and methanol (97:3 by volume) as eluent. The pure fractions were collected and the eluent was evaporated. The residue was crystallized from 2-propanol. The product was filtered off and dried, yielding 1 part (21%) of (E)-3-methoxy-6-[4-(3-phenyl-2-propenyl)-1-piperazinyl]-pyridazine; mp. 135.0°C (4).

Example 7

A mixture of 4.7 parts of 4-chloro-1-(3-chloro-1-propenyl)benzene, 4 parts of 3-chloro-6-(1-piperazinyl)pyridazine, 5.3 parts of sodium carbonate and 72 parts of N,N-dimethylformamide was stirred overnight at 65°C. The reaction mixture was evaporated. Water was added. The product was filtered off, washed with water and dissolved in trichloromethane. The organic layer was dried, filtered and evaporated. The residue was crystallized from a mixture of methanol and 2-propanol. The

product was filtered off and dried, yielding 4.5 parts (64%) of (E)-3-chloro-6-[4-[3-(4-chlorophenyl)-2-propenyl]-1-piperazinyl]pyridazine; mp. 177.9°C - (5).

In a similar manner there were also prepared:

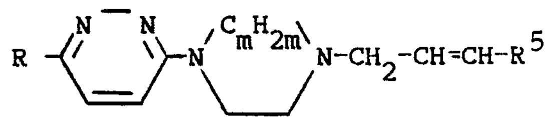

| No. | R | m | $R^5$ | isomeric form | base/ salt | mp.(°C) |
|---|---|---|---|---|---|---|
| 6 | $C_6H_5$- | 2 | $C_6H_5$- | E | base | 176.8 |
| 7 | Cl- | 3 | $C_6H_5$- | E | base | 130.9 |
| 8 | H- | 2 | $C_6H_5$- | E | 2HCl.1/2$H_2O$ | 225.2 |
| 9 | $CH_3O$- | 2 | 4-F-$C_6H_4$- | E | base | 143.1 |
| 10 | Cl- | 2 | 4-F-$C_6H_4$- | E | base | 165.7 |
| 11 | H- | 2 | 4-F-$C_6H_4$- | E | base | 127.1 |
| 12 | Cl- | 2 | 2-thienyl | E | base | 127.1 |
| 13 | Cl- | 2 | 4-$CH_3$-$C_6H_4$- | E | base | 164.0 |
| 14 | Cl- | 2 | 4-$CH_3O$-$C_6H_4$- | E | base | 146.8 |
| 15 | $C_6H_5$- | 3 | 2-thienyl | E | base | 124.9 |
| 16 | H | 2 | 4-$CH_3$-$C_6H_4$- | E | base | 125.2 |
| 17 | $C_6H_5$- | 2 | 4F-$C_6H_4$- | E | base | 197.3 |
| 18 | H | 2 | 4$CH_3O$-$C_6H_4$- | E | 2HCl.1/2$H_2O$ | 200.6 |
| 19 | $C_6H_5$- | 3 | $C_6H_5$- | E | base | 104.1 |

Example 8

A mixture of 6.9 parts of 3-phenyl-2-propynol methanesulfonate (ester), 6 parts of 3-chloro-6-(1-piperazinyl)pyridazine, 9.54 parts of sodium carbonate and 180 parts of N,N-dimethylformamide was stirred overnight at ±65°C. The reaction mixture was poured into ice water and the product was extracted with dichloromethane. The extract was dried, filtered and evaporated. The residue was crystallized from 2-propanol. The product was filtered off and dried, yielding 7.8 parts (83.1%) of 3-chloro-6-[4-(3-phenyl-2-propynyl)-1-piperazinyl]-pyridazine; mp. 123.2°C (20).

Example 9

A mixture of 3.5 parts of (E)-(3-chloro-1-propenyl)benzene, 4.8 parts of 3-chloro-6-(3-methyl-1-piperazinyl)pyridazine, 6.4 parts of sodium carbonate and 200 parts of 4-methyl-2-pentanone was stirred over weekend at reflux temperature using a water separator. After cooling, water was added and the layers were separated. The organic layer was dried, filtered and evaporated. The residue was purified by column chromatography over silica

gel using a mixture of trichloromethane and methanol (98.5:1.5 by volume) as eluent. The pure fractions were collected and the eluent was evaporated. The residue was crystallized from 2-propanol. The product was filtered off and dried, yielding 1 part (13.5%) of (E)-3-chloro-6-[3-methyl-4-(3-phenyl-2-propenyl)-1-piperazinyl]pyridazine; mp. 117.2°C (21).

In a similar manner there were also prepared:

(E)-3-chloro-6-[2-methyl-4-(3-phenyl-2-propenyl)-1-piperazinyl]pyridazine (22);

(E)-3-chloro-6-[2,3-dimethyl-4-(3-phenyl-2-propenyl)-1-piperazinyl]pyridazine (23);

(cis)-(E)-3-chloro-6-[2,5-dimethyl-4-(3-phenyl-2-propenyl)-1-piperazinyl]pyridazine monohydrochloride;mp. 123.0°C (24);

(E)-3-chloro-6-[3,5-dimethyl-4-(3-phenyl-2-propenyl)-1-piperazinyl]pyridazine; mp. 129.8°C; and

(E)-3-chloro-6-[2,6-dimethyl-4-(3-phenyl-2-propenyl)-1-piperazinyl]pyridazine (26);

Example 10

A solution of 7.2 parts of 3-phenyl-6-(1-piperazinyl)pyridazine in 200 parts of 2-propanol was acidified with 2-propanol, saturated with hydrogen chloride. 4.3 Parts of 1-phenylethanone and 0.9 parts of poly(oxymethylene) were added and the whole was stirred for 3 hours. Another portion of 0.9 parts of poly(oxymethylene) was added and stirring was continued for 8 hours at reflux. After the addition of 0.9 parts of poly(oxymethylene), the whole was stirred and refluxed over weekend. The product was filtered off and dried, yielding 6.9 parts (51.6%) of 1-phenyl-3-[4-(6-phenyl-3-pyridazinyl)-1-piperazinyl]-1-propanone dihydrochloride; mp. 191°C (27).

Following the same procedure and using equivalent amounts of the appropriate starting materials, there were also prepared:

3-[4-(6-chloro-3-pyridazinyl)-1-piperazinyl]-1-phenyl-1-propanone; mp. 138.2°C (28); and

3-[4-(6-chloro-5-methyl-3-pyridazinyl)-1-piperazinyl]-1-phenyl-1-propanone; mp. 131.3°C - (29).

Example 11

To a stirred mixture of 3 parts of 3-[4-(6-chloro-3-pyridazinyl)-1-piperazinyl]-1-phenyl-1-propanone and 80 parts of methanol were added portionwise 0.76 parts of sodium borohydride. Upon completion, stirring was continued for 20 hours at room temperature. The reaction mixture was evaporated. Water was added. The product was extracted with trichloromethane. The extract was dried, filtered and evaporated. The residue was crystallized from a mixture of 2-propanol and 2,2'-oxybispropane. The product was filtered off and dried, yielding 2.5 parts (83%) of 4-(6-chloro-3-pyridazinyl-α-phenyl-1-piperazinepropanol; mp. 144.5°C (30).

Following the same procedure and using equivalent amounts of the appropriate starting materials, there was also prepared:

α-phenyl-4-(6-phenyl-3-pyridazinyl)-1-piperazinepropanol; mp. 162.9°C (31).

Example 12

A mixture of 6.3 parts of 3-chloro-6-[4-(3-phenyl-2-propynyl)-1-piperazinyl]pyridazine, 3 parts of a solution of thiophene in methanol 4% and 120 parts of methanol was hydrogenated at normal pressure and at room temperature with 2 parts of platinum-on-charcoal catalyst 5%. After the calculated amount of hydrogen was taken up, the catalyst was filtered off and the filtrate was evaporated. The residue was purified by column chromatography over silica gel using a mixture of trichloromethane and acetonitrile (70:30 by volume) as eluent. The pure fractions were collected and the eluent was evaporated. The residue was crystallized from 2-propanol. The product was filtered off and dried, yielding 1.6 parts (25.4%) of (Z)-3-chloro-6-[4-(3-phenyl-2-propenyl)-1-piperazinyl]pyridazine; mp. 118.0°C (32).

Example 13

To a stirred solution of 1.2 parts of sodium in 30 parts of benzenemethanol were added 7.9 parts of (E)-3-chloro-6-[4-(3-phenyl-2-propenyl)-1-piperazinyl]pyridazine and stirring was continued for 6 hours at ±170°C. After cooling, water and dichloromethane were added. The separated organic layer was dried, filtered and evaporated. The residue was purified by column chromatography over silica gel using a mixture of trichloromethane and methanol (98.5:1.5 by volume) as eluent. The pure fractions were collected and the eluent was evaporated. The residue was crystallized from 2-propanol. The product was filtered off and dried, yielding 0.8 parts (10.7%) of (E)-6-[4-(3-phenyl-2-propenyl)-1-piperazinyl]-3(2H)pyridazinone; mp. 174.8°C (33).

Example 14

To a stirred solution of 3.93 parts of (E)-3-chloro-6-[4-(3-phenyl-2-propenyl)-1-piperazinyl]-pyridazine in 80 parts of 2-propanol was added a warm solution of 1.5 parts of (Z)-2-butenedioic acid in 40 parts of 2-propanone. The salt was filtered off and dried, yielding 5.3 parts (98.4%) of (E)-3-chloro-6-[4-(3-phenyl-2-propenyl)-1-piperazinyl]-pyridazine (Z)-2-butenedioate(1:1); mp. 179.8°C - (34).

In a similar manner there were also prepared:

(E)-3-chloro-6-[4-(3-phenyl-2-propenyl)-1-piperazinyl]pyridazine monohydrochloride; mp. 245.8°C (35);

(+)-(E)-3-chloro-6-[4-(3-phenyl-2-propenyl)-1-piperazinyl]pyridazine [R-(R*,R*)]-2,3-dihydroxybutanedioate(1:1).hemihydrate; mp. 131.9°C; $[\alpha]^{25}_{D}$ = + 9.67 (c = 1% in methanol) - (36); and

(E)-3-chloro-6-[4-(3-phenyl-2-propenyl)-1-piperazinyl]pyridazine dihydrochloride; mp. 249.7°C (37).

Example 15

To a stirred and cooled (ice bath, -15°C) solution of 10 parts of (E)-3-chloro-6-[4-(3-phenyl-2-propenyl)-1-piperazinyl]pyridazine in 300 parts of trichloromethane were added at once 5.5 parts of 3-chlorobenzenecarboperozoic acid. The mixture was allowed to reach room temperature during a period of 3 hours, cooled again and allowed to reach room temperature overnight. Another portion of 5.5 parts of 3-chlorobenzenecarboperoxoic acid were added while cooling and the mixture was allowed to reach room temperature. The mixture was washed twice with a sodium hydrogen carbonate solution. The organic layer was dried, filtered and evaporated at ±38°C. The residue was stirred for a few hours in 2-propanol. The precipitated product was filtered off and dissolved in a mixture of trichloromethane, methanol and ammonium hydroxide (90:10:1 by volume) and the mixture was filtered over silicagel, using the same mixture as eluent. The desired fractions were evaporated and the residue was boiled in 2,2'-oxybispropane. The product was filtered off and crystallized from acetonitrile. The product was filtered off and dried in vacuo, yielding 1.2 parts (11.3%) of (E)-3-chloro-6-[4-(3-phenyl-2-propenyl)-1-piperazinyl]pyridazine, 4-oxide; mp. 141.7°C (38).

C. Pharmacological Examples

The useful pharmacological properties of the compounds of formula (I) and their pharmacological acceptable acid-addition salts can be demonstrated by the "Tail Withdrawal Test" and by the "Acetic Acid-Induced Writhing in Rats Test".

Example 16

Tail Withdrawal Test

Male Wistar rats weighing 200g ± 5g were fasted overnight, water being available at libitum. Aqueous solutions, made isotonic with sodium chloride, of the test compounds at various concentrations were administered to the test animals intraveneously (i.v.). The thus treated rats were put into individual restraining cages. At various time periods after administration, the lower 5 cm portion of the tail was immersed into a cup filled with water at a constant temperature of 55°C. The typical tail withdrawal response was evaluated during a 10 sec. period after the immersion. $ED_{50}$ values in mg/kg body weight were determined as that dose of the test compound capable of suppressing in 50% of the tested animals the typical tail withdrawal response during a time period exceeding 10 seconds. Said $ED_{50}$ values for a number of compounds having the formula (I) and the pharmaceutically acceptable acid-addition salts thereof are gathered in Table 1.

Example 17

Acetic Acid-Induced Writhing in Rats Test.

The Acetic Acid-Induced Writhing in Rats Test is known to be a useful tool for the evaluation of the analgesic properties of given test compound - (see in connection herewith Drug Research, 25, 1505-1509 (1975)).

Method

Wistar rats weighing about 100g were used throughout. They were injected i.p. with 0.5 ml of a 1% aqueous solution of acetic acid and placed in individual glass containers for observation. Rats

exhibiting at least 10 writhes within 10 min. following the injection were selected. 5 Min. later (15 min. after the acetic acid treatment) the test compound was administered. 45 Min. later (60 min. after the acetic acid treatment), the animals were observed for 15 minutes, during which the number of writhes was counted.

The lowest effective dose in 50% of the test animals ($ED_{50}$) was determined as that dose in mg/kg body weight capable of reducing the number of writhes during the 15 minutes observation period to less than 16 writhes in 50% of the tested animals.

Said $ED_{50}$ values for a number of compounds having the formula (I) and the pharmaceutically acceptable acid-addition salts thereof are gathered in Table 1.

Table 1

| Comp. No. | Tail Witdrawal Test $ED_{50}$ in mg/kg body weight | Writhing in Rats Test $ED_{50}$ in mg/kg body weight |
|---|---|---|
| 1 | 20 | 5 |
| 3 | - | 10 |
| 4 | 40 | 5 |
| 5 | - | 40 |
| 7 | 40 | 20 |
| 8 | - | 2.5 |
| 9 | - | 10 |
| 10 | - | 20 |
| 11 | - | 40 |
| 12 | - | 10 |
| 13 | - | 40 |
| 14 | - | 40 |
| 18 | - | 40 |
| 19 | - | 10 |
| 37 | <40 | 10 |
| 2 | 10 | 5 |
| 38 | ≥20 | 10 |

## D) Composition Examples

The following formulations exemplify typical pharmaceutical compositions in dosage unit form suitable for systemic administration to animal and human subjects in accordance with the instant invention.

"Active ingredient" (A.I.) as used throughout these examples relates to a compound of formula -(I) or a pharmaceutically acceptable acid addition salt thereof.

### Example 18 : ORAL DROPS

500 g of the A.I. was dissolved in 0.5 l of 2-hydroxypropanoic acid and 1.5 l of the polyethylene glycol at 60~80°C. After cooling to 30~40°C there were added 35 l of polyethylene glycol and the mixture was stirred well. Then there was added a solution of 1750 g of sodium saccharin in 2.5 l of purified water and while stirring there were added 2.5 l of cocoa flavor and polyethylene glycol q.s. to a volume of 50 l, providing an oral drop solution comprising 10 mg of the A.I. per ml. The resulting solution was filled into suitable containers.

### Example 19 : ORAL SOLUTION

9 g of methyl 4-hydroxybenzoate and 1 g of propyl 4-hydroxybenzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propanetriol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution comprising 20 mg of the active ingredient per teaspoonful -(5 ml). The resulting solution was filled in suitable containers.

### Example 20 : CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelating capsules, comprising each 20 mg of the active ingredient.

### Example 21 : FILM-COATED TABLETS

Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90®) in about 200 ml of water. The wet powder mixture was sieved, dried and sieved again. Then there was added 100 g microcrystalline cellulose -(Avicel®) and 15 g hydrogenated vegetable oil -(Sterotex ®). The whole was mixed well and compressed into tablets, giving 10.000 tablets, each containing 10 mg of the active ingredient.

Coating

To a solution of 10 g methyl cellulose -(Methocel 60 HG®) in 75 ml of denaturated ethanol there was added a solution of 5 g of ethyl cellulose (Ethocel 22 cps ®) in 150 ml of dichloromethane. Then there were added 75 ml of dichloromethane and 2.5 ml 1,2,3-propanetriol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated colour suspension -(Opaspray K-1-2109®) and the whole was homogenated. The tablet cores were coated with the thus obtained mixture in a coating apparatus.

### Example 22 : INJECTABLE SOLUTION

1.8 g methyl 4-hydroxybenzoate and 0.2 g propyl 4-hydroxybenzoate were dissolved in about 0.5 l of boiling water for injection. After cooling to about 50°C there were added while stirring 4 g lactic acid, 0.05 g propylene glycol and 4 g of the A.I..

The solution was cooled to room temperature and supplemented with water for injection q.s. ad 1 l volume, giving a solution of 4 mg A.I. per ml. The solution was sterilized by filtration (U.S.P. XVII p. 811) and filled in sterile containers.

Example 23 : SUPPOSITORIES

3 g A.I. was dissolved in a solution of 3 g 2,3-dihydroxybutanedioic acid in 25 ml polyethylene glycol 400. 12 g Surfactant (SPAN®) and triglycerides (Witepsol 555 ®) q.s. ad 300 g were molten together. The latter mixture was mixed well with the former solution. The thus obtained mixture was poured into moulds at a temperature of 37~38°C to form 100 suppositories each containing 30 mg of the active ingredient.

**Claims**

1. A chemical compound having the formula

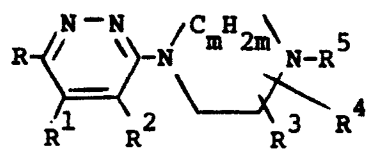

(I),

a N-oxide form, a pharmaceutically acceptable acid addition salt or a possible stereochemically isomeric form thereof, wherein R is hydrogen, halo, $C_{1-6}$alkyloxy, hydroxy or phenyl;

m is the integer 2 or 3;

$R^1$, $R^2$, $R^3$ and $R^4$ are, each independently, hydrogen or $C_{1-6}$alkyl;

or where $R^3$ and $R^4$ are substituted on a different carbon atom, $R^3$ and $R^4$ taken together, may form a bivalent radical $-CH_2-CH_2-$;

$R^5$ is (aryl)$C_{2-6}$alkenyl, (aryl)$C_{2-6}$alkynyl, (aryl)-(hydroxy)$C_{1-6}$alkyl or (aryl)(oxo)$C_{1-6}$alkyl;

wherein aryl is phenyl, being optionally substituted with up to 3 substituents, each independently selected from the group consisting of halo, $C_{1-6}$alkyl, trifluoromethyl, nitro, amino, $C_{1-6}$alkyloxy, hydroxy and $C_{1-6}$alkyloxycarbonyl; thienyl; and naphthalenyl.

2. A compound according to claim 1, wherein R is halo, $R^5$ is 3-aryl-2-propenyl, $R^1$, $R^2$, $R^3$ and $R^4$ are hydrogen and m is 2.

3. A chemical compound according to claim 1 wherein the compound is 3-chloro-6-[4-(3-phenyl-2-propenyl)-1-piperazinyl]pyridazine.

4. A pharmaceutical composition comprising a suitable pharmaceutical carrier and as active ingredient a therapeutically effective amount of a compound as claimed in any one of claims 1 to 3.

5. An analgesic pharmaceutical composition comprising a suitable pharmaceutical carrier and as active ingredient an effective analgesic amount of a compound as claimed in any one of claims 1 to 3.

6. A method of preparing a pharmaceutical composition, characterized in that a therapeutically effective amount of a compound as defined in any of claims 1 to 3 is intimately mixed with suitable pharmaceutical carriers.

7. A compound as claimed in any one of claims 1 to 3 for use as a medicine.

8. A compound as claimed in any one of claims 1 to 3 for use as an analgesic medicine.

9. A process for preparing a chemical compound as claimed in any one of claims 1 to 3, characterized by

a) N-alkylating an amine of formula

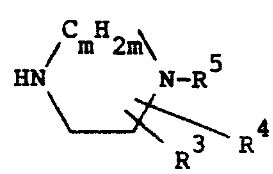

(II)

with a reagent of formula

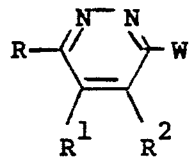

(III)

wherein W represents a reactive leaving group, in a reaction-inert solvent; or

b) N-alkylating an amine of formula

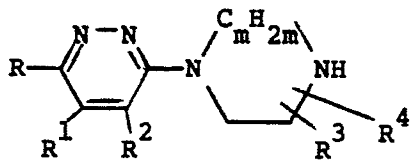

(IV)

with a reagent of formula

W-$R^5$ (V)

wherein W represents a reactive leaving group, in a reaction-inert solvent; or, optionally converting the compounds of formula (I) into each other following art-known group transformation procedures, and if desired, converting the compounds of formula (I) into the N-oxide form by treatment with a peroxide preferably in a reaction-inert solvent; and, if further desired, converting the compounds of formula (I) into a therapeutically active non-toxic acid addition salt form by treatment with an appropriate acid or; conversely, converting the acid addition salt into the free base form with alkali; and/or preparing stereochemically isomeric forms thereof.